## Europäisches Patentamt
## European Patent Office
## Office européen des brevets

(19)

(11) Veröffentlichungsnummer: **0 121 107**
**A1**

(12) ## EUROPÄISCHE PATENTANMELDUNG

(21) Anmeldenummer: **84102180.1**

(22) Anmeldetag: **01.03.84**

(51) Int. Cl.³: **A 23 J 3/00**
**C 12 N 1/00**

(30) Priorität: **07.03.83 DE 3308024**

(43) Veröffentlichungstag der Anmeldung:
**10.10.84 Patentblatt 84/41**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Scharf, Udo, Dr.**
**Altenhainer Strasse 20**
**D-6233 Kelkheim (Taunus(DE)**

(72) Erfinder: **Nöltner, Gerhard**
**Sossenheimer Weg 33a**
**D-6230 Frankfurt am Main 80(DE)**

(72) Erfinder: **Schlingmann, Merten, Dr.**
**Schneidhainer Strasse 32a**
**D-6240 Königstein/Taunus(DE)**

(54) **Verfahren zur Konditionierung mikrobieller Zellmassen.**

(57) Die Eigenschaften getrockneter mikrobieller Zellmassen werden durch eine Hitzebehandlung bei Temperaturen von 105 bis 160°C verbessert. Bei einer anschließenden Extraktion der Lipide und Nucleinsäuren zeigen die Verfahrensprodukte anwendungstechnische Vorteile sowie einen geringeren Proteinverlust bei höherer biologischer Wertigkeit.

EP 0 121 107 A1

HOECHST AKTIENGESELLSCHAFT   HOE 83/F 033     Dr.KL/mk     0121107

## Verfahren zur Konditionierung mikrobieller Zellmassen

Die Erfindung betrifft die Aufbereitung mikrobieller Zellmassen, um sie in eine zur Weiterverarbeitung besser geeignete Form zu überführen.

Mikrobielle Zellmassen sind wertvolle Proteinquellen, die jedoch für die menschliche Ernährung nicht ohne weiteres verwendbar sind. So müssen aus Bakterienzellmassen, die einen hohen Gehalt an Nucleinsäuren aufweisen, zur Vermeidung von Gesundheitsschädigungen die Nucleinsäuren weitgehend entzogen werden. Wegen des Geruchs und Geschmacks sowie aus Gründen der Haltbarkeit ist auch eine weitgehende Entfernung der Lipide notwendig. Zur Entfernung dieser störenden Bestandteile wurden bereits Extraktionsverfahren vorgeschlagen. Bei diesen Extraktionen tritt jedoch ein mehr oder weniger großer Feststoff- und damit verbunden ein Proteinverlust auf.

Es wurde nun gefunden, daß die Aufarbeitung mikrobieller Zellmassen erheblich erleichtert wird, wenn das Rohmaterial einer thermischen Behandlung vor der Extraktion der Lipide und Nucleinsäuren unterzogen wird.

Die Erfindung betrifft somit ein Verfahren zur Konditionierung mikrobieller Zellmassen, das dadurch gekennzeichnet ist, daß man die getrockneten Zellmassen einer Hitzebehandlung bei Temperaturen von 105 - 160°C unterwirft. Bevorzugte Ausgestaltungen dieser Erfindung sind im folgenden näher erläutert.

Die Dauer der Hitzebehandlung hängt vom Ausgangsmaterial, insbesondere seiner Restfeuchte, und selbstverständlich von der Behandlungstemperatur ab. Zweckmäßig ist eine Dauer von 3 - 40 Minuten, besonders vorteilhaft von 5 bis 20 Minuten in einem Produkt-Temperaturbereich von 105-140°C.

Die zum Einsatz kommende mikrobielle Rohbiomasse sollte einen Wassergehalt unter 10 Gew.-%, vorteilhaft von etwa 3 bis 5 Gew.-%, aufweisen, was nach den üblichen Produktschonenden Verfahren der Kontakttrocknung, insbesondere Walzentrocknung, oder Konvektionstrocknung wie Sprühtrocknung erreicht werden kann. Vorteilhaft sind getrocknete Rohmaterialien mit einem Schüttgewicht über 400 g/l, einem Rüttgewicht über 500 g/l und einer Korngrößenverteilung mit 75 % über 40 μm. Bevorzugt ist ein Rohprodukt, wie es nach dem Verfahren der DE-PS 26 33 451 erhalten wird.

Das Verfahren kann in allen üblichen Apparaten durchgeführt werden, in denen die thermische Energie durch Kontakt, Strahlung oder Konvektion mit Hilfe eines gasförmigen Mediums übertragen wird.

Die Verfahrensprodukte weisen eine Reihe von vorteilhaften Eigenschaften auf:

Die Verbesserung der mechanischen Eigenschaften zeigt sich nicht nur in einer erhöhten Haltbarkeit und Lagerbeständigkeit, sondern auch in einer leichteren Abfüll- und Dosierbarkeit, vor allem jedoch in einem verbesserten Verhalten bei einer anschließenden Extraktion von Lipiden und Nucleinsäuren. Diese nachfolgenden Verfahren erfordern mehrere fest-flüssig-Trennungen in Form von Filtration, Zentrifugation, Membrantrennverfahren und/oder Reversosmose. Überraschenderweise muß die Verbesserung der mechanischen Eigenschaften nicht mit einer Einbuße im Extraktionsverhalten erkauft werden, vor allem aber werden die physiologischen Eigenschaften des Endproduktes nicht nur nicht beeinträchtigt, sondern sogar signifikant verbessert.

Besonders vorteilhaft ist ein Aufarbeitungsverfahren nach der DE-PS 26 33 666, bei dem der Lipid- und Nucleinsäuregehalt der Zellmassen in einem zweistufigen Extraktionsverfahren vermindert wird. Zunächst werden die Zellmassen mit einer Extraktionsmischung aus Ammoniak und einem polaren Lösemittel aus der Reihe der niederen aliphatischen Alkohole, der niederen Glykole oder der niederen Alkylether eines niedermolekularen Glykols, die maximal 30 Gew.-% (bezogen auf die Lösemittelmenge) Wasser enthält, behandelt. Methanol wird als Lösemittel bevorzugt. Der Ammoniakgehalt beträgt vorteilhaft 1 - 10 Gew.-%, bezogen auf die Lösemittelmenge. In diesem ersten Schritt werden die Lipide extrahiert. Hierauf folgt ein zweiter Extraktionsschritt, bei dem die Nucleinsäuren mit Wasser weitgehend herausgelöst werden. Man erhält so etwa 60 bis 80 % der eingesetzten Rohmasse als Reinprodukt.

Wird vor diesem Extraktionsverfahren die erfindungsgemäße Konditionierung durchgeführt, so resultieren 10 bis 30 % höhere Feststoffausbeuten, ohne daß das Extraktionsergebnis hinsichtlich der Entfernung der Lipide und Nucleinsäuren beeinträchtigt wird. Zudem ergibt sich eine signifikante Verbesserung der biologischen Wertigkeit des Endproduktes.

Aus der DE-PS 27 45 954 ist ein Verfahren bekannt, bei dem ungereinigtes natürliches mikrobielles Protein in wäßrigem Medium einer Temperatur von etwa 40 bis 150°C bis zu erheblicher Proteinfällung ausgesetzt wird, worauf das ausgefällte Protein abgetrennt und enzymatisch abgebaut wird. Bei diesem Verfahren dient also die Erhitzung zur Fällung des Proteins aus seiner wäßrigen Lösung und damit zur Abtrennung von anderen, in Lösung verbleibenden Stoffen.

Aus der DE-OS 26 51 464 ist es bekannt, eine wäßrige Aufschlämmung von Mikrobenzellen bei relativ hohen Temperaturen und Drücken aufzubrechen. Bevorzugt werden Temperaturen von 60 bis etwa 200°C. Zur Zerstörung der Zellen erhitzt man bei 60°C auf etwa 20 Stunden und bei 200°C auf etwa 2 Stunden. Das freigesetzte Protein wird dann nach bekannten Verfahren von den Zellbruchstücken abgetrennt. Bei diesem Verfahren dient also das Erhitzen dazu, das Protein in wäßrige Lösung zu überführen.

Aus diesen bekannten Verfahren war somit nicht herleitbar, daß eine thermische Behandlung von trockener Bakterienzellmasse zu einem Produkt mit verbesserten verarbeitungstechnischen Eigenschaften führt, die sich in einem nachfolgenden Extraktionsverfahren vorteilhaft auswirken. Außerdem war nicht zu erwarten, daß die Verfahrensprodukte verbesserte ernährungsphysiologische Eigenschaften zeigen.

In den folgenden Beispielen wird die Erfindung näher erläutert.

Beispiel 1

Eine gemäß Beispiel 2 der DE-PS 26 33 451 erhaltene sprühgetrocknete Zellmasse mit 2 bis 4 % Restfeuchte, einem Schüttgewicht von 544 g/l, einem Rüttgewicht von 597 g/l und einer Korngrößenverteilung mit 90 %$>$40 ,um wird 30 Minuten in einem Wirbelbett bei 160$^{o}$C Lufttemperatur behandelt. Dabei wird 10 Minuten eine Produkttemperatur von 120$^{o}$C eingehalten. Das nach dem Abkühlen erhaltene Produkt zeigt bei mechanischer Beanspruchung einen deutlich verminderten Abrieb und eine verbesserte Lagerfähigkeit.

Werden 100 kg des so erhaltenen Produktes gemäß Beispiel 1 der DE-PS 26 33 666 extrahiert, erhält man 75 kg Reinprodukt mit einem Nucleinsäuregehalt von 1,5 Gew.-%, einem Fettgehalt von 0,8 Gew.-% und einem Proteingehalt von $>$90 %.

Das extrahierte Produkt weist also den gleichen Fett- und Nucleinsäuregehalt auf wie das Produkt gemäß Beispiel 1 der DE-PS 26 33 666, wobei jedoch die Proteinausbeute aufgrund der praktisch quantitativen Feststoffausbeute in den Extraktionsschritten um 15 % höher ist.

Beispiel 2

Bestimmung des PER-Werts (PER=protein efficiency ratio)

Verglichen wurde ein gemäß Beispiel 1 thermisch behandeltes und anschließend extrahiertes Produkt ("Erfindung") mit einem ohne thermische Vorbehandlung extrahierten Produkt ("Vergleich") hinsichtlich des PER-Wertes:

Die Prüfprodukte wurden auf Basis einer halbgereinigten Diät[*) verglichen, wobei sie Aminosäure-äquivalent auf Kosten von Maisstärke eingesetzt werden. Als Kontrolle wurde eine Diät mit Casein erstellt. Der zu prüfende Proteinträger bzw. Casein waren jeweils die einzige Proteinquelle in der Diät.

Die jeweilige Stickstoffkonzentration aus der Summe der Aminosäuren entsprach 10 % Reinprotein. Alle Diäten wurden mit DL-Methionin supplementiert. Als Versuchstiere dienten männliche Dawley-Ratten mit einem Durchschnittsgewicht von 70 g bei Versuchsbeginn. Pro Gruppe wurden 12 Tiere gewichtsmäßig so verteilt, daß gleichmäßige Anfangsgewichte erhalten wurden. Die Resultate zeigt die folgende Tabelle.

| Gruppe | Prüfprodukt | mittlere Gewichtszunahme in g nach 21 Tagen | | PER | |
| | | abs. | rel. | abs. | rel. |
| --- | --- | --- | --- | --- | --- |
| A | Casein | 136,7 | 100 | 3,56 | 100 |
| B | Erfindung | 156,8 | 114,7 | 4,46 | 125,3 |
| C | Vergleich | 136,5 | 99,9 | 3,82 | 107,2 |

-------

[*) GESELLSCHAFT FÜR ERNÄHRUNGSPHYSIOLOGIE DER HAUSTIERE, ARBEITSKREIS FÜR PROTEINBEWERTUNG, Vorschrift zur Proteinbewertung in Versuchen an wachsenden Ratten, Zeitschr. Tierphysiol. Tierern. Futtermittelkde., 19, (1964), 305-308.

**PATENTANSPRÜCHE:**

1. Verfahren zur Konditionierung mikrobieller Zellmassen, dadurch gekennzeichnet, daß man die getrockneten Zellmassen einer Hitzebehandlung bei Temperaturen von 105 bis 160°C unterwirft.

2. Verfahren nach Anspruch 1, dadurch gekennzeichnet, daß die Hitzebehandlung 3 bis 40 Minuten dauert.

3. Verfahren nach Anspruch 1 und 2, dadurch gekennzeichnet, daß die Hitzebehandlung 5 bis 20 Minuten bei Produkttemperaturen von 105-140°C durchgeführt wird.

4. Verfahren nach Anspruch 1 bis 3, dadurch gekennzeichnet, daß bei der Hitzebehandlung die thermische Energie durch Kontakt, Strahlung oder Konvektion mit Hilfe eines gasförmigen Mediums übertragen wird.

5. Konditionierte mikrobielle Zellmasse, erhältlich durch Hitzebehandlung einer getrockneten Zellmasse bei Temperaturen von 105 bis 160°C.

6. Verwendung der nach Anspruch 1 bis 4 erhaltenen konditionierten mikrobiellen Zellmassen bzw. der konditionierten Zellmassen nach Anspruch 5 als Ausgangsmaterial zur Extraktion von Lipiden und Nucleinsäuren bei der Herstellung von Lebensmitteln.

Patentansprüche für Österreich:

1. Verfahren zur Konditionierung mikrobieller Zellmassen, dadurch gekennzeichnet, daß man die getrockneten Zellmassen einer Hitzebehandlung bei Temperaturen von 105 bis 160°C unterwirft.

2. Verfahren nach Anspruch 1, dadurch gekennzeichnet, daß die Hitzebehandlung 3 bis 40 Minuten dauert.

3. Verfahren nach Anspruch 1 und 2, dadurch gekennzeichnet, daß die Hitzebehandlung 5 bis 20 Minuten bei Produkttemperaturen von 105-140°C durchgeführt wird.

4. Verfahren nach Anspruch 1 bis 3, dadurch gekennzeichnet, daß bei der Hitzebehandlung die thermische Energie durch Kontakt, Strahlung oder Konvektion mit Hilfe eines gasförmigen Mediums übertragen wird.

**Europäisches Patentamt**

# EUROPÄISCHER RECHERCHENBERICHT

EP 84 10 2180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A-2 210 662 (SOCIETE FRANCAISE DES PETROLES) * Ansprüche 1,7; Seite 2, Zeilen 34-38 * | 1-6 | A 23 J 3/00 C 12 N 1/00 |

-----

|  |  |  | RECHERCHIERTE SACHGEBIETE (Int. Cl. 3) |
|---|---|---|---|
|  |  |  | A 23 J C 12 B C 12 C C 12 N |

Der vorliegende Recherchenbericht wurde für alle Patentansprüche erstellt.

| Recherchenort DEN HAAG | Abschlußdatum der Recherche 15-07-1984 | Prüfer PEETERS J.C. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

 

& : Mitglied der gleichen Patentfamilie, übereinstimmendes Dokument

EPA Form 1503. 03.82